(19) Europäisches Patentamt
European Patent Office
Office européen des brevets

(11) Publication number: **0 416 691 A2**

(12) **EUROPEAN PATENT APPLICATION**

(21) Application number: **90202319.1**

(51) Int. Cl.5: **A61B 5/02**

(22) Date of filing: **31.08.90**

(30) Priority: **07.09.89 CH 3254/89**

(43) Date of publication of application: **13.03.91 Bulletin 91/11**

(84) Designated Contracting States: **CH DE ES FR GB IT LI**

(71) Applicant: **KONTRON INSTRUMENTS HOLDING N.V.**
**Pietermaai 6 A P.O. Box 504**
**Curacao(AN)**

(72) Inventor: **Eberhard, Patrick**
**Parkallee 11**
**CH-4123 Allschwil(CH)**
Inventor: **Palma, Jean Piere**
**Mühleweg 17**
**CH-4133 Pratteln(CH)**
Inventor: **Schollermann, Hans**
**Muttenzerstrasse 37**
**W-7889 Grenzach(DE)**

(74) Representative: **Riccardi, Sergio**
**Riccardi & Co. Via Macedonio Melloni, 32**
**I-20129 Milano(IT)**

# (54) Measuring sensor.

(57) The measuring sensor for the measurement of light absorption by blood in a part of the body, equipped with a light source and a light sensor possesses an angular support consisting of two flat wings, situated rigidly with respect to each other forming an angle of 90 - 170 degrees. A light source is situated in the first wing of the support and the light sensor in the other wing with both being in the surfaces facing each other.

7
α
2
4
5
6
3
1

EP 0 416 691 A2

Xerox Copy Centre

# MEASURING SENSOR

The invention relates to a measuring sensor for the measurement of light absorption by blood in a part of the body, equipped with a light source and a light sensor.

Such measurement sensors are used in diagnostic medicine for the determination of blood and circulation parameters, in particular for so-called oximetry. The measuring sensors of this type known today are described, for example, in the European Patent EP-B-O 127 947. Consequently there are essentially three types of measuring sensor, that is to say a first type in which the light source and the light sensor are opposite each other and the part of the body to be analysed is put between these. These measuring sensors are realised preferably in the form of a clamp. In another type of measuring sensor the light source and the light sensor are situated one beside the other on one plane. The light sensor receives the light from the light source flowing back from, or in, the part of the body. In a third form the light source and the light sensor are attached to a flexible support. In this form, according to the precise dimensions, the relative position of the light source and light sensor can vary from one beside the other to one opposite the other.

All the known measuring sensors have disadvantages. The clamp form is relatively complicated from a mechanical point of view and may be sized each time for only one given part of the body, for example a finger.

In the second form in which the light source and light sensor are placed one beside the other, there is a disadvantage because the signal is relatively weak and consequently the electronic structure for obtaining the signal must be notable. In the third form with the flexible support there is the problem of the mobility of the light source with respect to the light sensor. Even when this element is well fixed to the part of the body movement artefacts occur.

The present invention poses the problem of realising a measuring sensor of the above mentioned type which does not present the existing disadvantages of the known sensors and in particular, despite its mechanical simplicity, produces a utilisable, strong signal free of noise disturbance.

According to the present invention this is obtained with a measuring sensor of the above mentioned type characterised by an angular support consisting of two basically flat wings, that is to say planes or slightly concave or convex curves situated rigidly with respect to each other at an angle of 90 - 170 degrees, in which the light source is situated in one wing and the light sensor in the other wing with both being in the surfaces facing each other.

An example of the embodiment of the present invention is described hereinafter with reference to the attached diagrammatic drawing.

The measuring sensor essentially consists of a support or frame 1 which is in the form of an obtuse angle with two wings 2,3. The two wings are flat and of the same size and are united rigidly together forming an angle of 120 degrees. This angle $\alpha$ can vary according to its use and is within the range of 90 to 170 degrees appropriately. The support 1 preferably consists of synthetic material and is preferably moulded as one solid unit.

At the centre of the internal surface of the first wing 2 a luminous diode 4 is situated, the surface of which is flush with the surface of wing 2. At the centre of the internal surface of the other wing 3 a photosensor 5 is attached, the surface of which is similarly flush with the wing surface.

In correspondence with the side, there is a connecting cable 6 which contains the electric lines for the two active elements and serves for connection with the measuring apparatus 7.

The two active elements 4, 5 and their connecting lines as well as the extremity of the cable 6 are preferably embedded to the support 1 during manufacture.

The apparatus of the present invention is adapted for the measuring of oximetry for example. In this case on the other hand it is convenient to incorporate two or three luminous diodes in place of one only, with different wavelengths since the oximetric measurements are carried out with different wavelengths.

To carry out a measurement, the measuring sensor is preferably fixed with an adhesive tape to the appropriate part of the body, for example to a finger.

In the tests of the measuring sensor described above it was evident that this is largely insensitive with respect to movement and despite the more simplified production system it provides a signal substantially better than measuring sensors with simple reflection, in which the light source and sensor are situated next to each other in one plane.

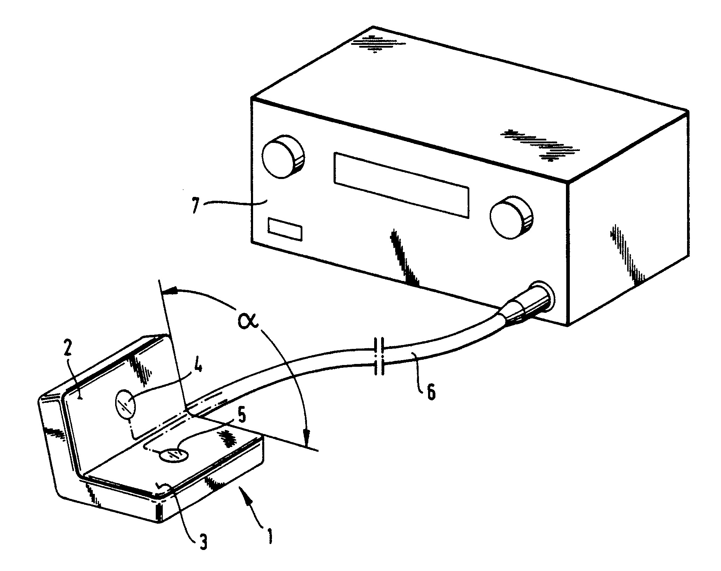

## Claims

Measuring sensor for the measurement of light absorption by blood in a part of the body, equipped with a light source and a light sensor characterised by an angular support consisting of two wings, situated rigidly with respect to each other forming an angle of 90 - 170 degrees (preferably 120 degrees) in which the light source is situated in one wing of the support and the light sensor in the other wing with both being in the surfaces facing each other.

7
α
2
4
5
6
3
1